# EUROPEAN PATENT APPLICATION

(11) **EP 4 763 144 A1**
(43) Date of publication of application: **24.06.2026**
(21) Application number: 25221332.7
(22) Date of filing: 08.12.2025
(51) Int. Cl.: A61F 2/30, A61B 17/72, A61F 2/36

(54) **FLEXIBLE STEM**

(30) Priority: 19.12.2024 US 202463736086 P
(71) Applicant: Waldemar Link GmbH & Co. KG, 22339 Hamburg (DE)
(72) Inventor: BAUER, Eckhard, 24159 Kiel (DE); LINK, Helmut D., 22339 Hamburg (DE)
(74) Representative: Alatis

(57) **Abstract**

The present disclosure includes a flexible stem (100) of a medical implant for implantation into a bone. The stem includes a stem shaft (101) having a first stem shaft end (102) and a second stem shaft end (104); and a plurality of projections (106), each of the plurality of projections having a first projection end (108) and a second projection end (110), wherein the first projection end of each of the plurality of projections is connected to the stem shaft.

## Description

### CROSS REFERENCE TO RELATED APPLICATIONS

This application claims priority from prior-filed U.S. Provisional Patent Application No. 63/736,086, filed December 19, 2024; the disclosure of which is incorporated herein by reference in its entirety.

### BACKGROUND OF THE DISCLOSURE

The present disclosure relates to stems of prosthetic of a medical implant for implantation into a bone. More particularly, the present disclosure relates to stems of prosthetics that are flexible and to use of the same.

Joint arthroplasty is a surgical procedure for replacing damaged components of a joint with prosthetic components. Such damage may be caused by, for example, traumatic injury or some form of arthritis, such as osteoarthritis. The replacement of joints, such as the shoulder, hip, knee, ankle and wrist, with prosthetic implants has become a relatively common practice.

For example, the hip joint is formed between the head of the femur and the acetabulum of the pelvis. Therefore, arthroplasty of the hip joint can involve replacing the femoral head with a prosthetic implant, which involves resecting the femoral head along its neck and preparing the intramedullary canal of the femur to receive a prosthetic, elongated intramedullary femoral stem.

Typically, these stems, such as the intramedullary femoral stem, are rigid and are formed of a stiff material, such as stainless steel. However, these typical rigid stems provide no flexibility or elasticity during implantation and can damage or cause undesirable pressures to the remaining bone.

Also, these typical rigid stems provide no flexibility or elasticity after implantation, during typical activities, which does not match the flexible/elastic properties of the natural bone and may cause damage or cause undesirable pressures to the remaining bone.

Therefore, what is desired is a stem that is at least partially flexible, the shape of which can be modified in response to various forces during implantation of the prosthetic and after the surgery is complete.

### SUMMARY OF THE DISCLOSURE

In accordance with one or more embodiments, devices and methods are provided.

The present disclosure includes a flexible stem of a medical implant for implantation into a bone. The stem includes a stem shaft having a first stem shaft end and a second stem shaft end; and a plurality of projections, each of the plurality of projections having a first projection end and a second projection end, wherein the first projection end of each of the plurality of projections is connected to the stem shaft.

### BRIEF DESCRIPTION OF THE DRAWINGS

The accompanying drawings, which are incorporated in and constitute a part of this specification, illustrate embodiments of the disclosure, and, together with the summary given above, and the detailed description of the embodiments below, serve as a further explanation and disclosure to explain and/or illustrate embodiments of the disclosure.
FIGs. 1A and 1B are perspective views of an embodiment of a stem of the present disclosure;
FIG. 2 is a vertical cross section of the stem of FIGs. 1A and 1B;
FIG. 3 is a cross-sectional view of options of a horizontal cross section of a stem of the present disclosure;
FIG. 4A-4C are vertical cross sections of stems of embodiments of the present disclosure;
FIGs. 5A and 5B are perspective views of an embodiment of a stem of the present disclosure.
FIG. 6 is a vertical cross section of the stem of FIGs. 5A and 5B;
FIG. 7 is a perspective views of the stem of FIGs. 5A and 5B, with one projection removed for illustrative purposes;
FIGs. 8A and 8B are perspective views of an embodiment of a stem of the present disclosure;
FIGs. 9A and 9B are perspective views of an embodiment of a stem of the present disclosure; and
FIG. 9C is a vertical cross section of the stem of FIGs. 9A and 9B.

### DETAILED DESCRIPTION OF THE DISCLOSURE

It is noted that the drawings of the present application are provided for illustrative purposes only and, as such, the drawings are not drawn to scale. It is also noted that like and corresponding elements are referred to by like reference numerals.

In the following description, numerous specific details are set forth, such as particular structures, components, materials, dimensions, processing steps and techniques, in order to provide an understanding of the various embodiments of the present application. However, it will be appreciated by one of ordinary skill in the art that various embodiments of the present application may be practiced without these specific details. In other instances, well-known structures or processing steps have not been described in detail in order to avoid obscuring the present application.

As used herein, the term "substantially" or "substantial", is equally applicable when used in a negative connotation to refer to the complete or near complete lack of an action, characteristic, property, state, structure, item, or result. For example, a surface that is "substantially" flat would either be completely at, or so nearly flat that the effect would be the same as if it were completely flat.

As used herein, terms defined in the singular are intended to include those terms defined in the plural and vice versa.

As used in this specification and its appended claims, terms such as "a", "an" and "the" are not intended to refer to only a singular entity but include the general class of which a specific example may be used for illustration, unless the context dictates otherwise. The terminology herein is used to describe specific embodiments of the disclosure, but their usage does not delimit the disclosure, except as outlined in the claims.

Unless otherwise indicated, all numbers expressing quantities of ingredients, properties such as molecular weights, reaction conditions, and so forth as used in the specification and claims are to be understood as being modified in all instances by the term "about." Accordingly, unless indicated to the contrary, the numerical parameters in the specification and claims are approximations that may vary depending upon the desired properties sought to be obtained by the present disclosure. At the very least, and without limiting the application of the doctrine of equivalents to the scope of the claims, each numerical parameter should at least be construed in light of the number of reported significant digits and by applying ordinary rounding techniques. Notwithstanding that the numerical ranges and parameters describing the broad scope of the disclosure are approximations, the numerical values in the specific examples are reported as precisely as possible. Any numerical value, however, inherently contains standard deviations that necessarily result from the errors found in the numerical value's testing measurements.

Thus, reference herein to any numerical range expressly includes each numerical value (including fractional numbers and whole numbers) encompassed by that range. To illustrate, reference herein to a range of "at least 50" or "at least about 50" includes whole numbers of 50, 51, 52, 53, 54, 55, 56, 57, 58, 59, 60, etc., and fractional numbers 50.1, 50.2 50.3, 50.4, 50.5, 50.6, 50.7, 50.8, 50.9, etc. In a further illustration, reference herein to a range of "less than 50" or "less than about 50" includes whole numbers 49, 48, 47, 46, 45, 44, 43, 42, 41, 40, etc., and fractional numbers 49.9, 49.8, 49.7, 49.6, 49.5, 49.4, 49.3, 49.2, 49.1, 49.0, etc. In yet another illustration, reference herein to a range of from "5 to 10" includes whole numbers of 5, 6, 7, 8, 9, and 10, and fractional numbers 5.1, 5.2, 5.3, 5,4, 5,5, 5.6, 5.7, 5.8, 5.9, etc.

In the discussion and claims herein, the tern "about" indicates that the value listed may be somewhat altered, as long as the alteration does not result in nonconformance of the process or structure to the illustrated embodiment. For example, for some elements the term "about" can refer to a variation of ±0.1%, for other elements, the term "about" can refer to a variation of ±1% or ±10%, or any point therein.

As used herein, the term "flexible," or any derivative thereof, is a broad term used in its ordinary sense and describes, for example, the ability of a component to bend, expand, contract, fold, unfold, or otherwise substantially deform or change shape. The term "flexible," or any derivative thereof, generally refers to a material being capable of bending or flexing such that it is pliant and yieldable in response to a change in surrounding condition (e.g., an external force or pressure). A flexible material can generally alter geometric shape and/or volume to accommodate a change in surrounding condition and/or to conform to the shape of an object brought in contact with it. The term "flexible," or any derivative thereof, includes continuously flexible components (such as a rubber tube, which bends at each point along its length) and constrained series of short, discrete links that allow the links to approximate the movement of a continuously flexible unit (such as a series "snake-like" vertebrae).

In some embodiments, stems of the present disclosure can be elastic. As used herein, the terms "elastic", "elastomeric", "elasticity" or derivations thereof are used to describe the ability of various materials and/or structures to reversibly undergo deformation under stress, e.g., become stretched or extended, in at least one direction when a force is applied to the material and/or structure and to resume substantially to their original dimensions upon relaxing, i.e., when the force is released, without (or with minimal) rupture or breakage. "Elastic" can refer to a material or combination of materials, and/or a structure or a combination of structures, that can be elongated in at least one direction by at least about 5% (or at least about 10%, or at least about 15%, or at least about 20%, or at least about 25%, or at least about 30%, etc.) of its relaxed, original length (*i.e.,* can stretch up to about 5%, or up to about 10%, up to about 15%, or up to about 20%, or up to about 25%, or up to about 30%), without rupture or breakage, and upon release of the applied force, recovers at least about 40% of its elongation. For example, a structure that has an initial length of about 100 mm can extend at least to about 110 mm, and upon removal of the force would retract to a length of about 106 mm (~40% recovery). "Elastic" may refer to a single material, or it may refer to a combination of materials forming an article. An elastic material structure may be incorporated and/or combined with other less elastic or not elastic material structures.

Reference now will be made in detail to embodiments of the disclosure. It will be apparent to those skilled in the art that various modifications and variations can be made in the present disclosure without departing from the scope or spirit of the disclosure. For instance, features illustrated or described as part of one embodiment can be used on another embodiment to yield a still further embodiment. As another example, features of each embodiment translate to the other embodiments, with combinations of embodiments and the descriptions thereof, within the skill of the art, included in this disclosure.

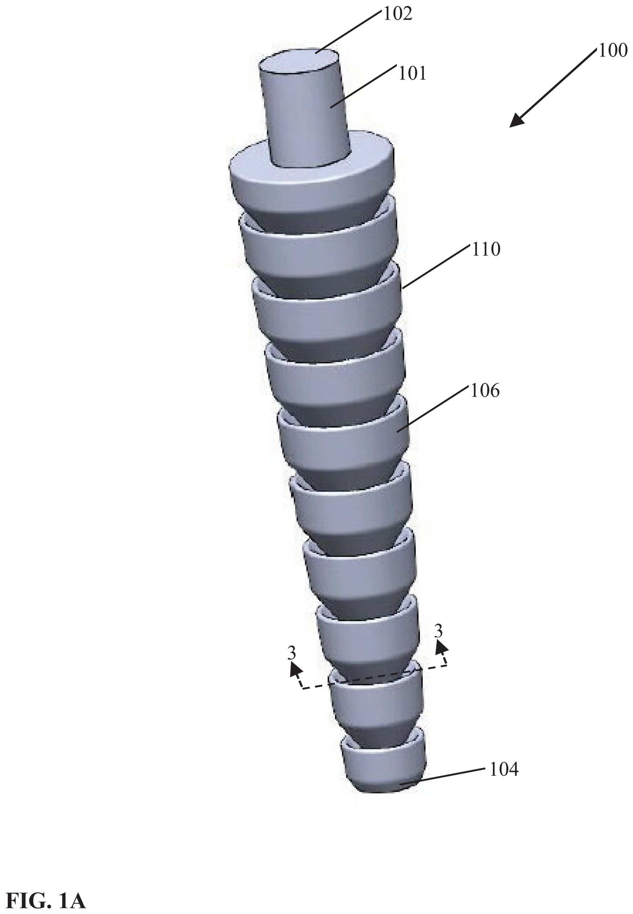

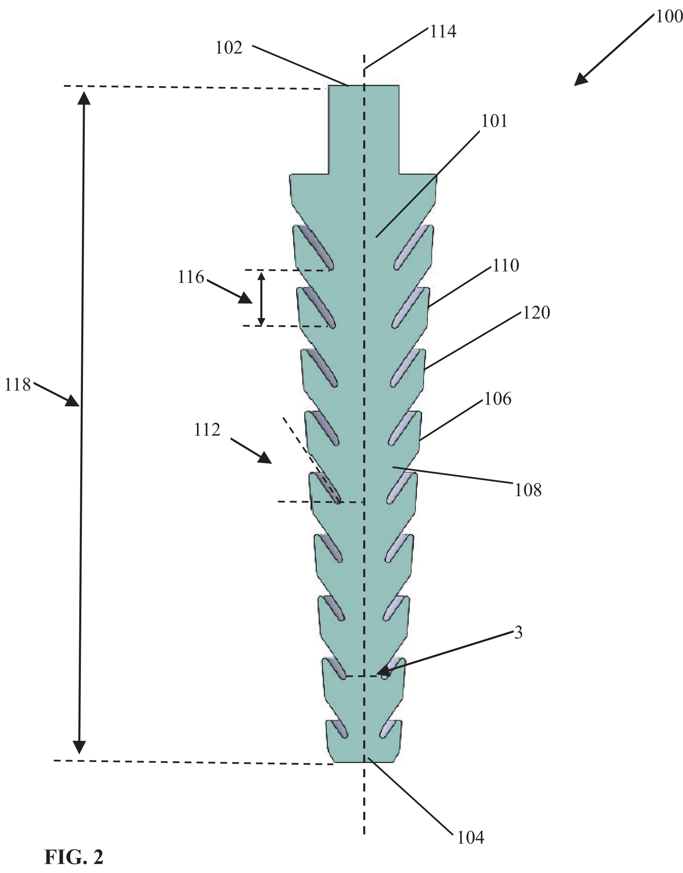

An embodiment of a flexible stem 100 of a medical implant for implantation into a bone of the present disclosure is shown in FIG. 1A. The stem 100 includes a stem shaft 101 having a first stem shaft end 102 and a second stem shaft end 104. The stem 100 also includes a plurality of projections 106. Each of the plurality of projections 106 comprising a first projection end (108 shown in FIG. 2) and a second projection end 110. The first projection end of each of the plurality of projections 106 is connected to the stem shaft 101.

The first stem shaft end 102, as well as any stem shaft of any embodiment of the present disclosure, is configured to connect to a medical and/or prosthetic connection, such as a femoral ball or humeral ball. In other embodiments the first stem shaft end 102 is configured to connect to any suitable medical and/or prosthetic connection. Similarly, the first stem shaft end of each embodiment is configured to connect to any suitable medical and/or prosthetic connection.

The second stem shaft end 104, as well as any stem shaft of any embodiment of the present disclosure, is configured to be inserted into a cavity formed within an intramedullary canal of a bone of a mammal. The bone of the mammal can be any bone with an intramedullary canal, such as shoulder bone(s), hip and/or pelvic bones, knee and/or leg bones, ankle, wrist, and/or arm bones, finger bones and/or hand bones, toe and/or foot bones, jaw and/or any suitable skull bones, vertebrae, etc. The stem 100, as well as all other stems in the present disclosure, can be placed into the cavity and maintained by pressure as a press-fit. Alternatively, or in addition to, a cement can be added to contact both the stem 100, etc. and the bone of implantation.

Any portion of the stem 100, as well as all stems of the present disclosure, can be flexible due to the design, material, orientation, and/or configuration etc. of that portion Alternatively, all portions of the stem 100, as well as all stems of the present disclosure, can be flexible due to the design, material, orientation, and/or configuration etc. of that portion.

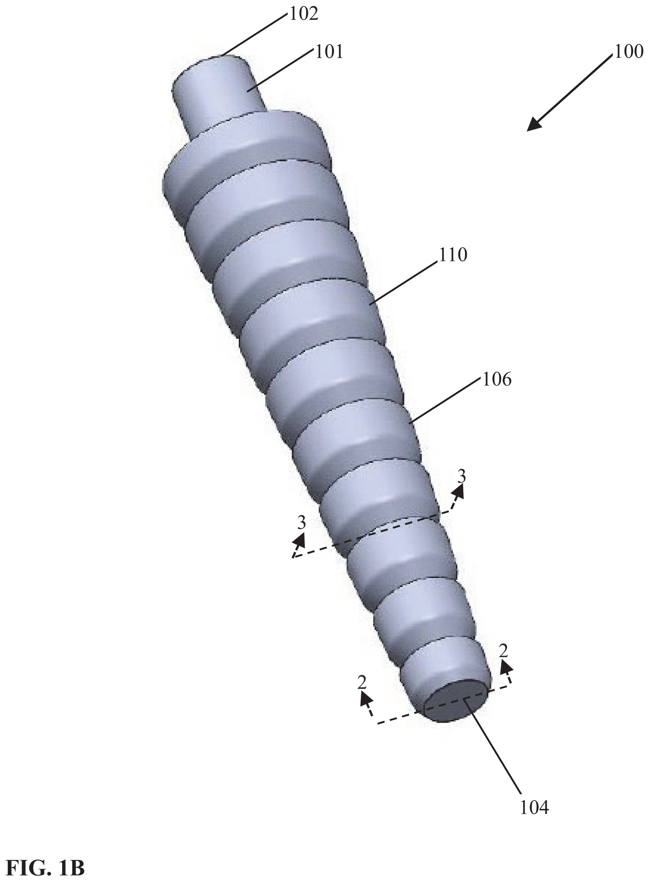

FIG. 1B illustrates a different perspective view of the stem 100 of FIG. 1A.

FIG. 2 is a vertical cross section of the stem 100 of FIGs. 1A and 1B. In this view of FIG. 2 the first projection end 108 is shown connected to the stem shaft 101. This connection, as well as any other connections in the present disclosure, can be any suitable mechanical connection, such as welding, etc., and/or this connection can be due to a unitary construction of the each of the plurality of projections 106 and the stem shaft 101. This unitary construction can be the result of any suitable manufacturing method, such as a milling method from a single piece of material that forms both the plurality of projections 106 and the stem shaft 101 and/or this unitary construction can be the result of any suitable additive manufacturing (AM) and/or 3D-printing technology. For example, the plurality of projections 106 and the stem shaft 101can be formed according to a suitable electron beam melting (EBM) process and/or a suitable selective laser melting (SLM) process.

EBM is an additive process for manufacturing and may produce solid or porous material. A powder of the desired material is provided in the desired granulometry. By the EBM process the powders of the desired material are deposited in successive layers at desired positions and in desired sequence (as defined in a preceding modelling step for the porous structure) and made to melt such as to form a coherent, solid body, such as any portion of, or the entirety of, any porous, implantable structure 100 disclosed herein.

SLM is a manufacturing technique that uses a laser to fuse metallic or non metallic powders, to form a portion, or the entirety of, the plurality of projections 106 and/or the stem shaft 101. The laser selectively fuses powdered material by scanning cross-sections generated from a 3-Dimensional digital description of the portion, or the entirety of, the plurality of projections 106 and/or the stem shaft 101, for example from a programmed computer file, on the surface of a powder bed. After each cross-section is scanned, the powder bed is lowered by one layer thickness, a new layer of material is applied on top, and the process is repeated until the portion, or the entirety of the plurality of projections 106 and the stem shaft 101 is manufactured to the desired degree.

As one example of this AM technology, the plurality of projections 106 and/or the stem shaft 101 can be additively manufactured from Ti-6Al-4V extra low interstitial (ELI) powder in an electron beam machine (Arcam EBM Q10 plus) to arrive at at least a partially flexible stem 100.

The present stem 100, as well as all other stems of the present application, is at least partially flexible. This flexible property can be due to the material of the stem 100, such as the plurality of projections 106 and/or the stem shaft 101 can be formed of any suitable material, such as Ag, Cu, Ni, Fe, Co, Cr, Au, Sn, Al, Zr, Li, Mn, Mg, Ti, Ir, Zn, Pt, and Ta, alloys thereof that can include non-metallic elements such as carbon, and combinations thereof. These materials can be biocompatible and may be partially or wholly coated in any suitable single or multiple coatings. This material can be varied as per design criteria. The material of the stem 100 can be consistent or substantially consistent along a height dimension 118 of the stem shaft 101, as it is in FIG. 2. In other embodiments the material can vary along the height dimensions 118, for example the material can be more flexible nearer the first stem shaft end 102 as compared to a different, less flexible material nearer the second stem shaft end 104.

As can be seen in FIG. 2, the stem 100 includes a plurality of projections 106. In this embodiment, nine projections 106 are shown, in other embodiments, two, three, four, five, six, seven, eight, ten, eleven, twelve, thirteen, fourteen, fifteen or more projections 106 can be included.

For the flexible stem 100, the first projection end 108 of each of the plurality of projections 106 is connected to the stem shaft 101 at a position vertically offset (shown in FIG. 2 as vertical offset distance 116), along the height dimension 118 of the stem shaft 101, from an adjacent first projection end 108. This vertical offset distance 116 can be varied as per design criteria. This vertical offset distance 116 can be consistent or substantially consistent along the height dimension 118, as it is in FIG. 2. In other embodiments the vertical offset distance 116 can vary along the height dimensions 118, for example the vertical offset distance 116 can be lesser nearer the first stem shaft end 102 as compared to the vertical offset distance 116 nearer the second stem shaft end 104.

As can be seen in both FIGs. 1A and 1B, as well as FIG. 2, the first projection end 108 of each of the plurality of projections 106 is connected to the stem shaft 101 along an entire circumference of the stem shaft 101. In other embodiments, the first projection end 108 of each of the plurality of projections 106 can be connected to the stem shaft 101 along at least a portion of a circumference of the stem shaft 101.

In this embodiment a projection angle 112, which is an angle from an axis substantially perpendicular to a stem axis 114 can be about 70° or greater, about 65° or greater, about 60° or greater, about 55° or greater, about 50° or greater, about 45° or greater, about 40° or greater, about 35° or greater, about 30° or greater, about 25° or greater, about 20° or greater, about 15° or greater, about 10° or greater, or about 5° or greater. This projection angle 112 can be varied as per design criteria. This projection angle 112 can be consistent or substantially consistent along the height dimension 118, as it is in FIG. 2. In other embodiments the projection angle 112 can vary along the height dimensions 118, for example the projection angle 112 can be lesser nearer the first stem shaft end 102 as compared to the projection angle 112 nearer the second stem shaft end 104. If a force is applied to any of the plurality of projections 106, the projection angle 112 can be altered due the flexibility of the projection 106. For example, upon application of a force, the projection 106 can bend towards the first stem shaft end 102 and the projection angle 112 can increase, and upon removal of that force, the projection 106 can substantially return to the configuration of FIG. 2 and the projection angle 112 can substantially return to the angle of FIG. 2.

As seen in FIG. 2, as well as in FIGs. 1A and 1B, the second projection end 110 comprises an external surface 120. This external surface 120 is substantially parallel to the stem axis 114. In this embodiment the stem axis 114 is substantially straight, in other embodiments the stem axis 114 can be angled and/or curved. Similarly, any stem axis of the present disclosure can be angled and/or curved. The external surface 120 can be configured to contact an interior of the bone surface upon implantation of the stem 100, similarly, any exterior surface of any stem of the present disclosure can be similarly configured as external surface 120.

The external surface 120 can comprise a porous surface structure, which can have an advantageous effect on the mechanical anchoring of the stem 100 through bone ingrowth and influence of the integration between the external surface 120 with bone tissue. The porous material of the external surface 120 can be formed by "basic" elements, wherein a basic element is shaped like, for example, a tetrapod, but in other embodiments any suitable shape can be formed. A tetrapod is a structure having four legs being connected at a center point, each of the legs pointing away from the center point and spanning with their free ends, for example a tetrahedron, but in other embodiments different shapes can be formed. In one embodiment, the porous material of the external surface 120 can be the porous structure TrabecuLink^{®}. In other embodiments, the porous material of the external surface 120 can be formed into pores of any suitable shape, such as circular pores, substantially circular pores, rectangular pores, substantially rectangular pores, oval pores, substantially oval pores, any suitable polyhedron shape pore, an erratic shape pore, etc.

FIG. 2 illustrates stem 100 in a non-implanted state. In an implanted state, where the stem 100 has been placed within a medullary canal of a patient's bone, some, most or all of the plurality of projections 106 will be contacting the bone surface. Each of the plurality of projections 106 are independently configured to bend towards from the stem axis 114 or bend away from the stem axis 114 in response to a force exerted by the bone during implantation of the stem 100 or while the stem 100 implanted. This bending of portions of the stem 100 is advantageous and can cause stem 100 to be more adaptable to the specific bone of the specific patient the stem 100 is implanted into.

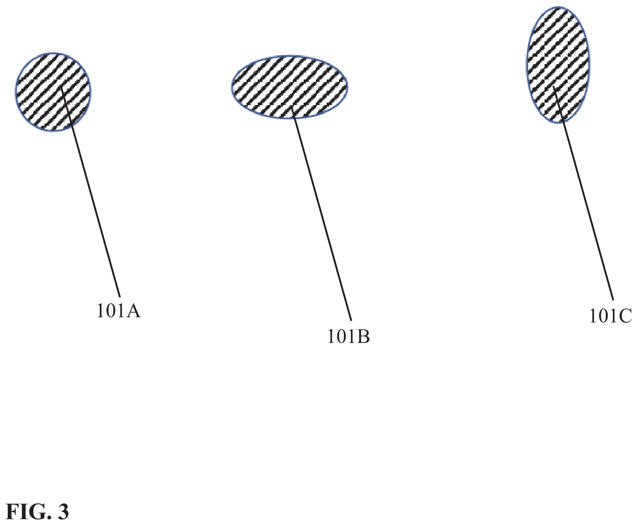

Cross-sectional views of the stem shaft 101 are shown in FIG. 3, with the locations indicated in FIGs. 1A, 1B and 2. In FIG. 3, three different options for the cross-sectional shape of the stem shaft 101 are shown as 101A, 101B and 101C. These three examples are shown, but in other embodiments, any suitable shape, size (including polygons and erratic shapes) can be included. The cross-sectional shape of the stem shaft 101 can be varied as per design criteria. This cross-sectional shape of the stem shaft 101 can be consistent or substantially consistent along the height dimension 118, as it is in FIG. 2. In other embodiments the cross-sectional shape of the stem shaft 101 can vary along the height dimensions 118. One example of this variation is that the diameter of the circular or ovular cross-section can be smaller nearer the first stem shaft end 102 as compared to diameter nearer the second stem shaft end 104, and vice versa. Another example of this variation is that the shape of the cross-section can change, such as the cross-section being circular nearer the first stem shaft end 102 and ovular nearer the second stem shaft end 104, and vice versa.

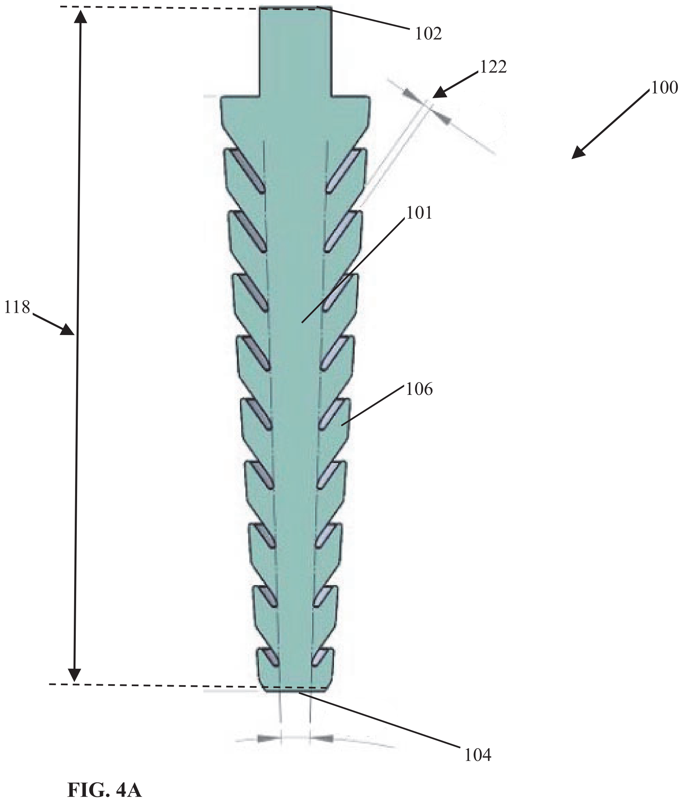

Examples of shafts 100 are shown in FIGs. 4A-4C. As can be seen in FIG. 4A, a diameter of the stem shaft 101 nearer the second stem shaft end 104 is smaller than a diameter of the stem shaft 101 nearer the first stem shaft end 102.

Additionally, in FIG. 4A a gap 122 between adjacent projections 106 is shown as one distance, in other embodiments this gap 122 can be any suitable size and can be fixed or variable along the height direction 118. The orientation of FIG. 4A creates a stem 100 that is less flexible towards the first stem shaft end 102 as compared to towards the second stem shaft end 104.

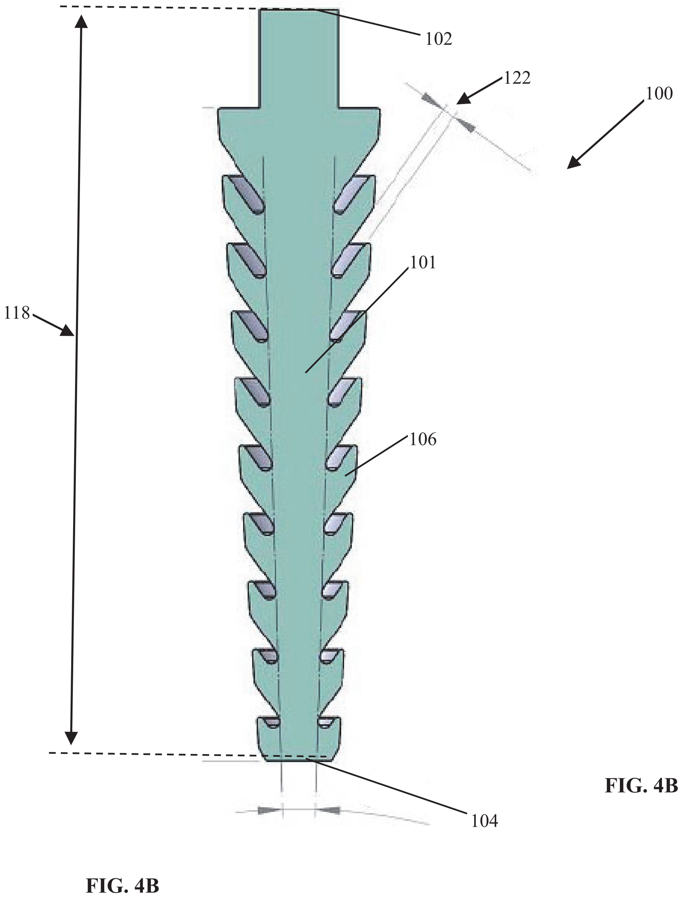

As can be seen in FIG. 4B, a diameter of the stem shaft 101 nearer the second stem shaft end 104 is smaller than a diameter of the stem shaft 101 nearer the first stem shaft end 102, which is similar to the configuration illustrated in FIG. 4A. In FIG. 4B the gap 122 between adjacent projections 106 is wider as compared to FIG. 4A. Thus, the orientation of FIG. 4B creates a stem 100 that is less flexible towards the first stem shaft end 102 as compared to towards the second stem shaft end 104 (like FIG. 4A), and also weaker as compared to FIG. 4A due to the thickness of the gap 122 and resultant lesser material.

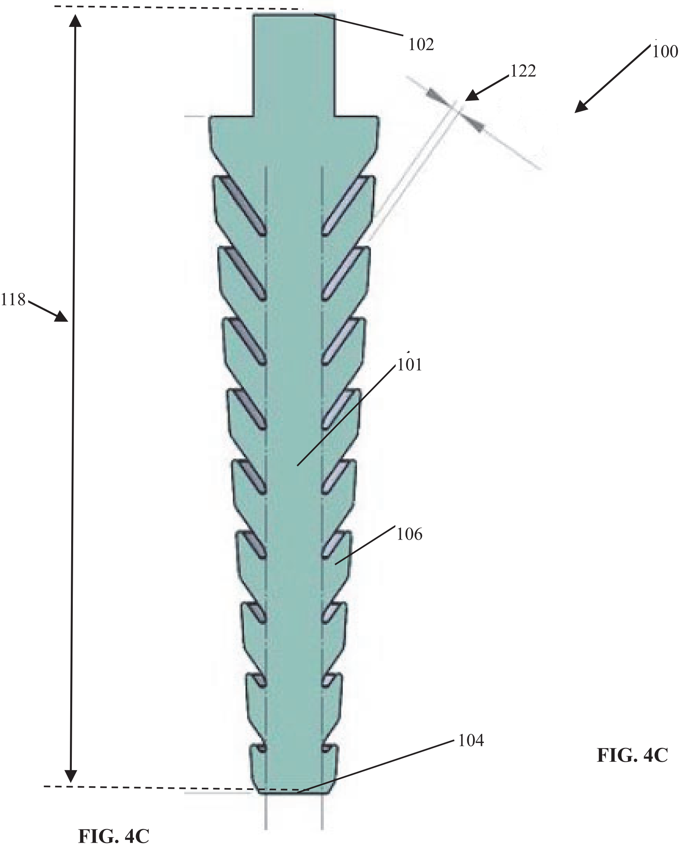

As can be seen in FIG. 4C, a diameter of the stem shaft 101 between the second stem shaft end 104 and the first stem shaft end 102 is substantially constant. Thus, the orientation of FIG. 4C creates a stem 100 that is substantially equally flexible along the height direction 118.

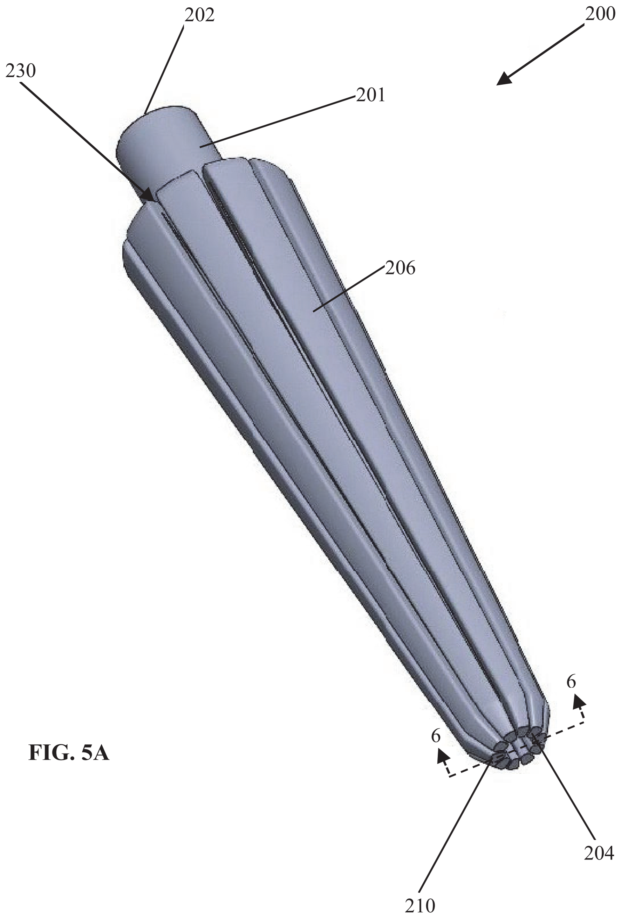

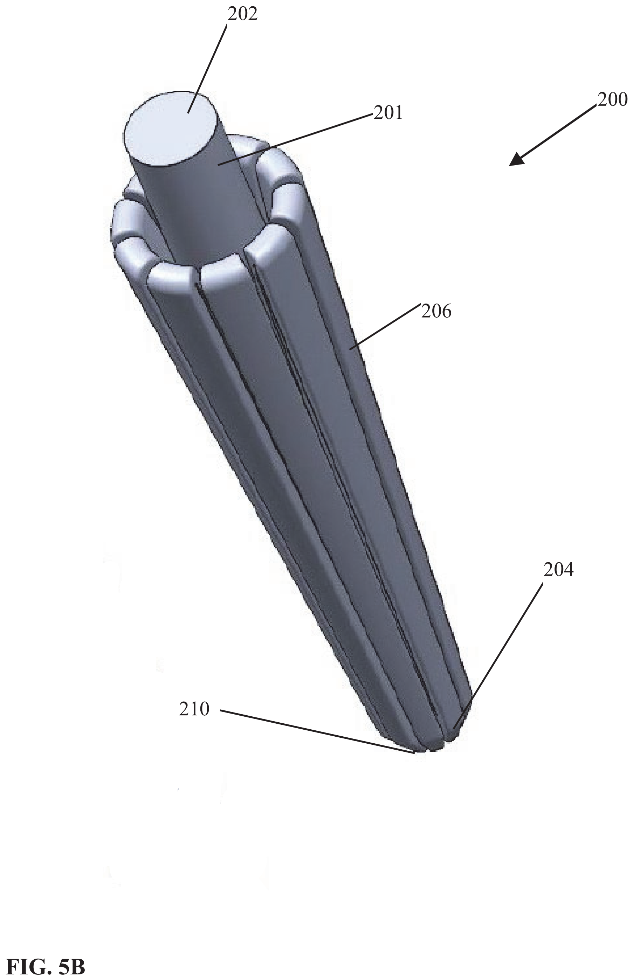

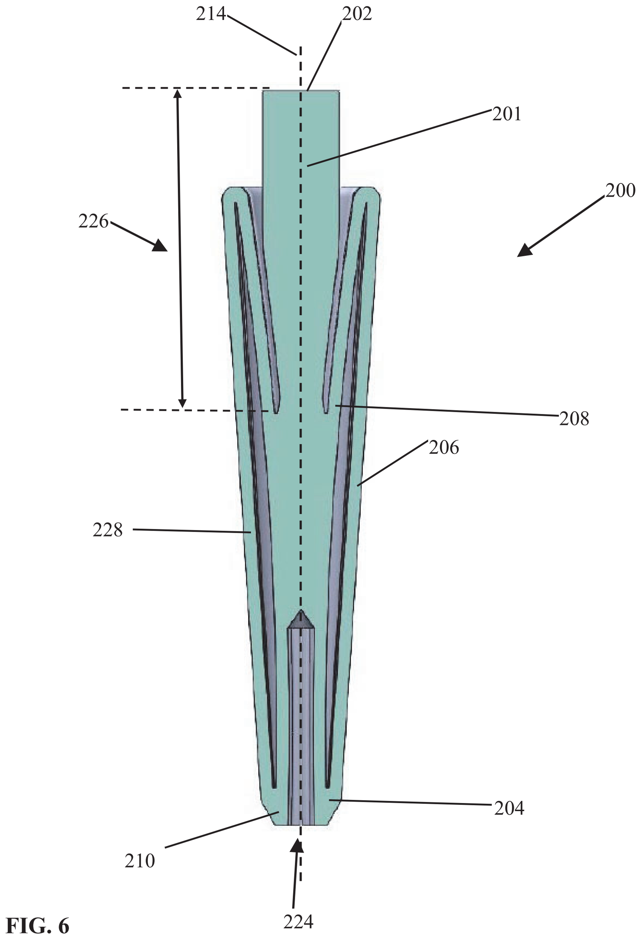

Another embodiment of a shaft 200 is shown in FIG. 5A and 5B, which are perspective views of an embodiment of the disclosure. FIGs. 5A and 5B illustrate an flexible stem 200 of a medical implant for implantation into a bone. The stem includes a stem shaft 201, the stem shaft 201 having a first stem shaft end 202 and a second stem shaft end 204. The stem 200 also includes a plurality of projections 206, each of the plurality of projections comprising a first projection end (208, shown in FIG. 6) and a second projection end 210, wherein the first projection end (208, shown in FIG. 6) of each of the plurality of projections 206 is connected to the stem shaft 201.

FIG. 6 is a vertical cross sectional view of the stem 200 of FIGs. 5A and 5B. As can be seen from the view of FIG. 6, the first projection end 208 is connected to the stem shaft 201. In this embodiment, the first projection end 208 is connected to the stem shaft 201 at a position vertically away, a distance 226 from the first stem shaft end 202, in other embodiments the distance 226 can be larger, smaller or no distance at all, with the first projection end 208 connected to the first stem shaft end 202.

An optional cavity 224 is shown in the vicinity of the second stem shaft end 204, which can aid in handling and/or implantation of the stem 200. In other embodiments this optional cavity 224 is not included and the region is substantially flat.

In this embodiment, the second projection end 210 of each of the plurality of projections is connected to the stem shaft 201.

For stem 200, as best seen in FIGs. 5A and 5B, the first projection end 208 of each of the plurality of projections 206 is connected to the stem shaft 201 at a position circumferentially offset, along a circumference near the first end 202 of the stem shaft 201, from an adjacent first projection end 208. Additionally, stem 200 includes the second projection end 210 of each of the plurality of projections 206 being connected to the stem shaft 201 at a position circumferentially offset, along a circumference near the second end 204 of the stem shaft 201, from an adjacent second projection end 210.

In the embodiment of stem 200 shown in FIGs. 5A and 5B, the plurality of projections 206 are connected to the stem shaft 201 around an entire circumference of the stem shaft 201. In other embodiments, the plurality of projections 206 are connected to a portion of the circumference of the stem shaft 201.

In the embodiment of stem 200 shown in FIGs. 5A and 5B the stem 100 includes, wherein adjacent projections 206 of the plurality of projections 206 are separated by a gap 230 that extends between the first projection end 208 and the second projection end 210, wherein the gap 230 is substantially parallel with a stem axis 214.

As seen in FIG. 6, a portion 228 of each of the plurality of projections 206 is a distance away from the stem shaft 201 between the first projection end 208 and the second projection end 210. Each of the plurality of projections 206 are independently configured to bend towards from a stem axis 214 or bend away from the stem axis 214, or bend towards an adjacent gap 230, in response to a force exerted by the bone during implantation of the stem 200 or while the stem 200 implanted. This bending can cause the portion 228 to get closer to or further from the stem axis 214, and/or closer or further from an adjacent projection 206. This bending of the portion 228 of the stem 200 is advantageous and can cause stem 200 to be more adaptable to the specific bone of the specific patient the stem 200 is implanted into.

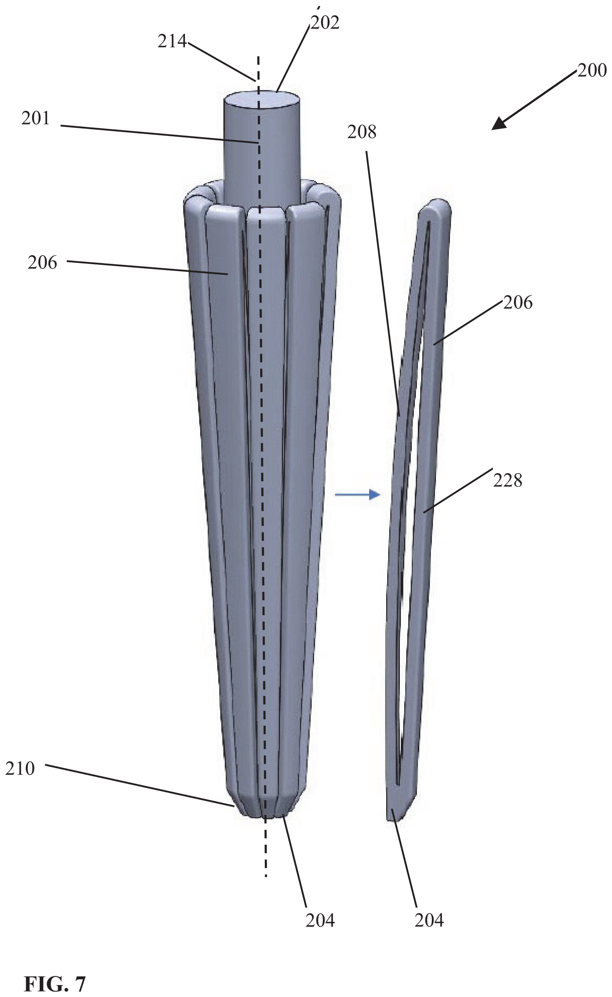

FIG. 7 is a perspective views of the stem 200 of FIGs. 5A and 5B, with one projection 206 removed for illustrative purposes. In this illustration the single projection 206 is not connected to the stem shaft 201 and is shifted away from the stem shaft 201 for illustrative purposes.

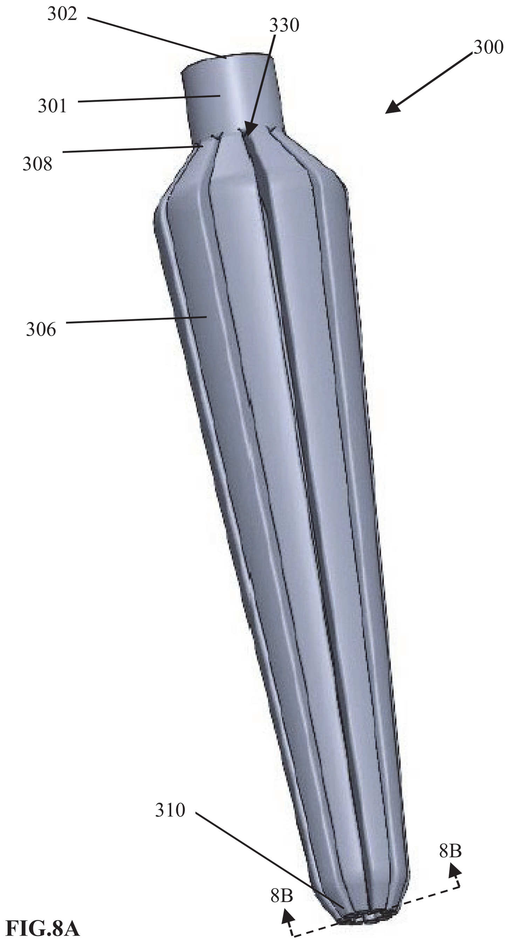

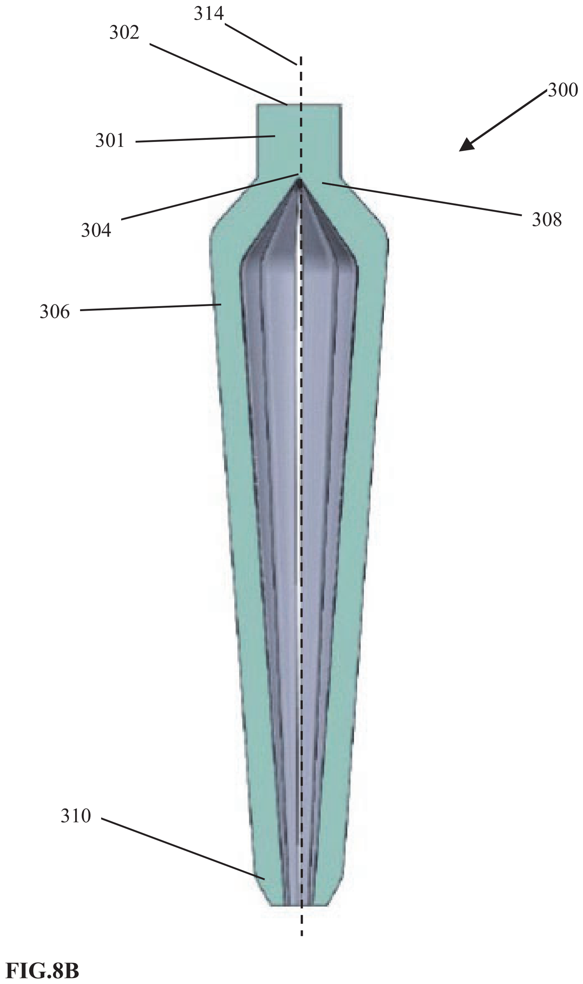

Another embodiment of a shaft 300 is shown in FIG. 8A, which is a perspective view of an embodiment of the disclosure. FIG. 8A illustrates a flexible stem 300 of a medical implant for implantation into a bone. The stem 300 includes a stem shaft 301, the stem shaft 301 having a first stem shaft end 302 and a second stem shaft end (304, shown in FIG. 8B). The stem 300 also includes a plurality of projections 306, each of the plurality of projections comprising a first projection end 308 and a second projection end 310, wherein the first projection end 308 of each of the plurality of projections 306 is connected to the stem shaft 201.

FIG. 8B is a vertical cross sectional view of the stem 300 of FIG. 8A. As can be seen from the view of FIG. 8B, the first projection end 308 is connected to the stem shaft 201, near the second stem shaft end 304. In this embodiment, the first projection end 308 is connected to the stem shaft 301 at or near the second stem shaft end 304, in other embodiments, the second stem shaft end 304 can extend past where the connection to the first projection end 308 is.

As can be seen in FIGs. 8A and 8B, for stem 300, the first projection end 308 of each of the plurality of projections 306 is connected to the stem shaft 301 at a position circumferentially offset, along a circumference near the second end 304 of the stem shaft 301, from an adjacent first projection end 308. In this embodiment, the second projection end 310 of each of the plurality of projections 306 is not connected to the stem shaft 301 and may or may not be connected to adjacent second projection ends 310.

In the embodiment of the stem 300 as shown in FIG. 8A, adjacent projections 306 of the plurality of projections 306 are separated by a gap 330 that extends between the first projection end 308 and the second projection end 310, wherein the gap 330 is substantially parallel with a stem axis 314.

As seen in FIG. 8A and 8B, each of the plurality of projections 306 is a distance away from the stem axis 314. Each of the plurality of projections 306 are independently configured to bend towards from a stem axis 314 or bend away from the stem axis 314, or bend towards an adjacent gap 330, in response to a force exerted by the bone during implantation of the stem 300 or while the stem 300 implanted. This bending can cause the projection 306 to get closer to or further from the stem axis 314, and/or closer or further from an adjacent projection 306. This bending of one or more of the plurality of projections 306 is advantageous and can cause stem 300 to be more adaptable to the specific bone of the specific patient the stem 300 is implanted into.

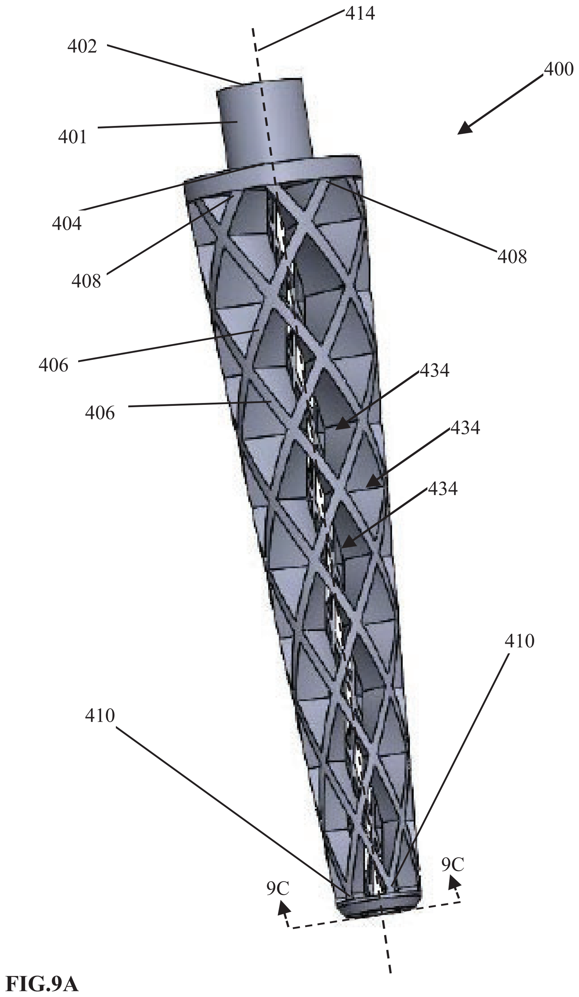

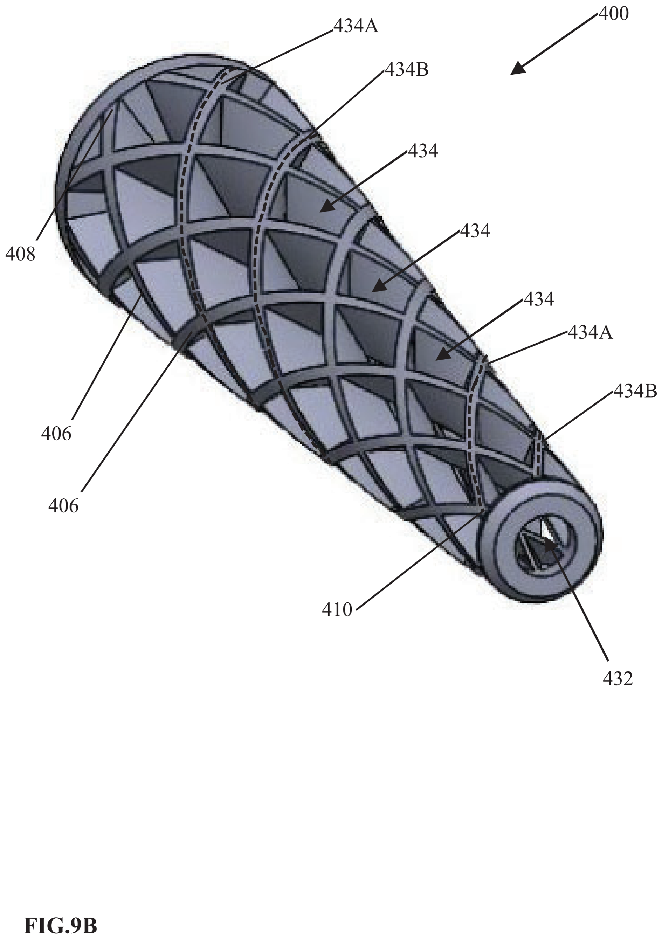

Another embodiment of a shaft 400 is shown in FIGs. 9A and 9B, which are perspective views of an embodiment of the disclosure. FIGs. 9A and 9B illustrate a flexible stem 400 of a medical implant for implantation into a bone. The stem 400 includes a stem shaft 401, the stem shaft 401 having a first stem shaft end 402 and a second stem shaft end 404. The stem 400 also includes a plurality of projections 406, each of the plurality of projections comprising a first projection end 408 and a second projection end 410, wherein the first projection end 408 of each of the plurality of projections 406 is connected to the stem shaft 401.

An optional cavity 432 is shown in FIG. 9B, which can aid in handling and/or implantation of the stem 200. In other embodiments this optional cavity 432 is not included and the region is substantially flat.

As can be seen in both FIGs. 9A and 9B, each of the plurality of projections 406 intersects another of the plurality of projections, one or more times, between the first projection end 408 and the second projection end 410. The intersection angle of the plurality of projections 406 can be altered from the about 30° angle shown in FIG. 9A and 9B, to a larger or smaller angle, such as about 75° or greater, about 70° or greater, about 65° or greater, about 60° or greater, about 55° or greater, about 50° or greater, about 45° or greater, about 40° or greater, about 35° or greater, about 25° or greater, about 20° or greater, about 15° or greater, about 10° or greater, or about 5° or greater, to modify the flexibility of the stem 400.

A plurality of openings 434 are formed between adjacent projections 406. These openings 434 can optionally include, or be filled with, a porous material, which can be the same material or a similar material to the material of the external surface 120 of stem 100 discussed herein.

As best seen in FIG. 9B, each of the plurality of projections 406 comprises a projection distance 434 (434A as one example, 434B as a second example of FIG. 9B), between the first projection end 408 and the second projection end 410. Due the helical shape of each of the plurality of projections 406, the projection distance 434 is longer than a height of the stem shaft 401 and also longer than a height between the first projection end 408 and the second projection end 410.

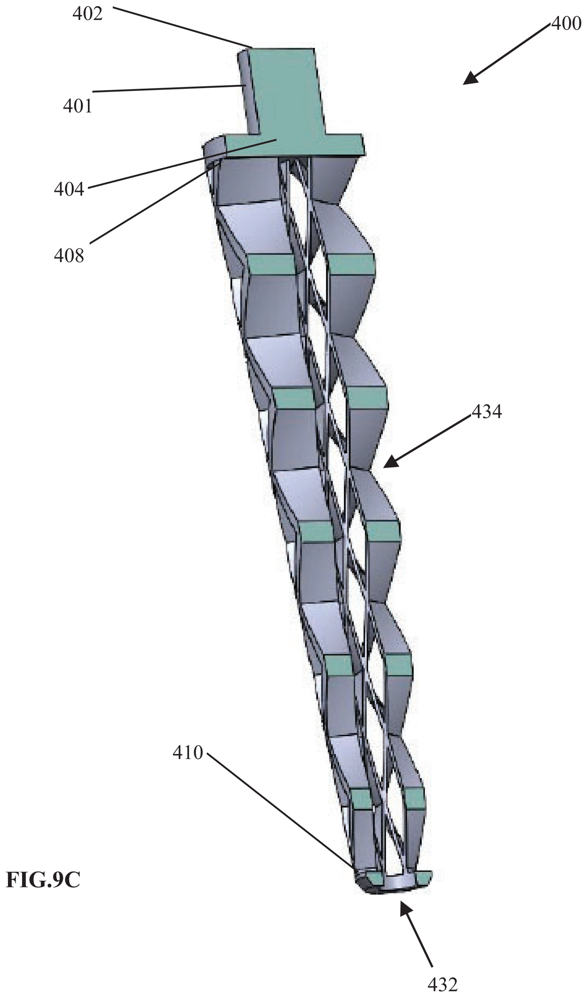

FIG. 9C is a vertical cross section of the stem of FIGs. 9A and 9B.

The described embodiments and examples of the present disclosure are intended to be illustrative rather than restrictive and are not intended to represent every embodiment or example of the present disclosure. While the fundamental novel features of the disclosure as applied to various specific embodiments thereof have been shown, described and pointed out, it will also be understood that various omissions, substitutions and changes in the form and details of the devices illustrated and, in their operation, may be made by those skilled in the art without departing from the spirit of the disclosure. For example, it is expressly intended that all combinations of those elements and/or method steps which perform substantially the same function in substantially the same way to achieve the same results are within the scope of the disclosure. Moreover, it should be recognized that structures and/or elements and/or method steps shown and/or described in connection with any disclosed form or embodiment of the disclosure may be incorporated in any other disclosed or described or suggested form or embodiment as a general matter of design choice. Further, various modifications and variations can be made without departing from the spirit or scope of the disclosure as set forth in the following claims both literally and in equivalents recognized in law.

## Claims

1. A flexible stem of a medical implant for implantation into a bone, the stem comprising:
a stem shaft comprising a first stem shaft end and a second stem shaft end; and
a plurality of projections, each of the plurality of projections comprising a first projection end and a second projection end, wherein the first projection end of each of the plurality of projections is connected to the stem shaft.

2. The flexible stem of claim 1, wherein the first projection end of each of the plurality of projections is connected along at least a portion of a circumference of the stem shaft.

3. The flexible stem of any of the preceding claims, wherein the first projection end of each of the plurality of projections is connected along an entire circumference of the stem shaft.

4. The flexible stem of any of the preceding claims, wherein the first projection end of each of the plurality of projections is connected to the stem shaft at a position vertically offset, along a height dimension of the stem shaft, from an adjacent first projection end.

5. The flexible stem of any of the preceding claims, wherein the second projection end comprises an external surface, wherein the external surface is substantially parallel with a stem axis.

6. The flexible stem of any of the preceding claims, wherein a second end stem shaft thickness is less than a first end stem shaft thickness.

7. The flexible stem of any of the preceding claims, wherein a second end stem shaft thickness is more than a first end stem shaft thickness.

8. The flexible stem of any of the preceding claims, wherein each of the plurality of projections is independently configured to bend towards or bend away from a stem axis in response to a force.

9. The flexible stem of any of the preceding claims, wherein the second projection end of each of the plurality of projections is connected to the stem shaft.

10. The flexible stem of any of the preceding claims, wherein the first projection end of each of the plurality of projections is connected to the stem shaft at a position vertically offset from the first end.

11. The flexible stem of any of the preceding claims, wherein the first projection end of each of the plurality of projections is connected to the stem shaft at a position circumferentially offset, along a circumference near the first end of the stem shaft, from an adjacent first projection end.

12. The flexible stem of any of the preceding claims, wherein adjacent projections of the plurality of projections are separated by a gap that extends between the first projection end and the second projection end, wherein the gap is substantially parallel with a stem axis.

13. The flexible stem of any of the preceding claims, wherein each of the projections comprises an internal surface and an external surface, wherein at least a portion of the external surface of at least one of the plurality of projections comprises a porous structure.

14. The flexible stem of any of the preceding claims, wherein the second projection end of each of the plurality of projections is not connected to the stem shaft and is not connected to adjacent second projection ends.

15. The flexible stem of any of the preceding claims, wherein each of the plurality of projections intersects another of the plurality projections between the first projection end and the second projection end.
